# DEMANDE DE BREVET EUROPEEN

(11) **EP 0 534 028 A1**
(43) Date de publication de la demande: **31.03.1993**
(21) Numéro de dépôt: 91402584.6
(22) Date de dépôt: 27.09.1991
(51) Int. Cl.: C12Q 1/68, C12Q 1/70, C07K 17/12, G01N 33/53, G01N 33/537, G01N 33/571

(54) **Procédé de fixation d'une séquence nucléotidique sur un support solide utilisant un anticorps**

(71) Demandeur: APPLIGENE, F-67402 Illkirch Cédex (FR)
(72) Inventeur: Tchen, Paul, F-92000 Nanterre (FR); Vautherot, Jean-François, F-78610 Auffargis (FR)
(74) Mandataire: Ayache, Monique

(57) **Abrégé**

L'invention a pour objet un procédé de fixation d'une séquence nucléotidique sur un support poreux par l'intermédiaire d'un anticorps, faisant d'une part, intervenir l'adsorption de l'anticorps sur le support et d'autre part, une réaction de type immunologique spécifique entre un substituant de la séquence nucléotidique, ou la séquence nucléotidique hybridée avec une molécule complémentaire, et ledit anticorps, caractérisé en ce que l'on mélange d'abord une solution de l'anticorps et une solution de la séquence nucléotidique substituée ou hybridée et on fait passer ensuite le mélange, après incubation ou non, à travers le support poreux.

Application, entre autres, au typage d'agents pathogènes, de préférence après amplification de la ou des séquence(s) nucléotidique(s) caractéristique(s).

## Description

L'invention concerne la fixation de séquences nucléotidiques au niveau d'un support.

Elle concerne plus particulièrement la fixation de séquences nucléotidiques courtes ou oligonucléotides sur des supports solides et son application à la détection et/ou au dosage de gènes particuliers dans un mélange de séquences nucléotidiques.

L'invention fournit un procédé général de fixation d'un acide nucléique, en particulier d'un oligonucléotide, sur un support solide par l'intermédiaire d'un anticorps et de sa réaction avec une molécule pour laquelle il présente une affinité.

Les acides nucléiques sont des molécules linéaires pouvant s'apparier en structures hybrides antiparallèles complémentaires. Cette propriété est mise à profit lorsqu'on utilise les acides nucléiques comme sondes pour détecter des séquences de nucléotides, séquences qui déterminent l'information génétique portée par ces molécules.

Des tests basés sur l'utilisation des sondes nucléiques peuvent être appliqués par exemple à l'étude des agents pathogènes viraux ou cellulaires, à la détection des mutations impliquées dans les maladies génétiques ou la modification de cellules tumorales, à la caractérisation d'allèles du complexe majeur d'histocompatibilité etc.

Ces tests comportent généralement une étape de fixation de la molécule à étudier (la cible) sur un support solide, puis la mise en présence de cette molécule fixée avec une molécule libre (la sonde) dans un milieu liquide approprié (le tampon d'hydridation) permettant la formation d'hydrides stables entre molécules complémentaires. Les molécules sondes, dont la séquence est connue, sont marquées de telle sorte qu'elles puissent être détectées après l'étape d'hybridation et l'élimination des molécules non hybridées sur les molécules cibles. L'inverse est également possible : fixation des molécules portant la séquence connue, et hybridation avec des molécules du spécimen à étudier, après marquage de ces dernières.

Les procédés utilisant la fixation d'une des molécules sont les plus commodes, car ils permettent d'éliminer facilement les molécules libres marquées non hybridées par simple rinçage du support portant les molécules fixées. Les supports couramment utilisés [membranes poreuses de nitrocellulose, de nylon ou de difluorure de polyvinylidène (PVDF)] ont la propriété d'adsorber les acides nucléiques, mais cette propriété ne s'applique qu'à des molécules assez longues (plusieurs centaines de nucléotides).

Les développements récents des techniques de génétique moléculaire conduisent à utiliser des molécules de plus en plus courtes dans les tests utilisant des sondes nucléiques. En effet, le but de tels tests est généralement de détecter des mutations ponctuelles ou des différences minimes au niveau des séquences des gènes. Une telle spécificité est aujourd'hui possible en utilisant comme sondes des désoxyribonucléotides synthétiques courts. De plus, il est possible d'augmenter enzymatiquement, par "PCR" (Polymerase Chain Reaction), la quantité de molécules à analyser (R.K. Saiki et coll., Science vol 230, 1985, p 1350-1354). Dans ce type de réaction enzymatique, il est avantageux de n'amplifier que la région génomique strictement nécessaire à l'analyse à effectuer, d'une part, parce qu'ainsi une même quantité de molécules amplifiées pourra être obtenue plus rapidement et avec moins de substrat, d'autre part, parce qu'en limitant la taille de la cible, on limitera les risques d'hybridations non spécifiques et de "bruit de fond".

Pour pouvoir pleinement exploiter les possibilités offertes par ces techniques reposant sur l'utilisation d'acides nucléiques courts, il est nécessaire de trouver un moyen de fixer aisément des oligonucléotides sur des supports solides.

On a déjà proposé des techniques permettant d'immobiliser des séquences nucléotidiques sur des supports, notamment après amplification de la séquence à analyser. Des exemples de ces techniques sont résumés dans ce qui suit.

Ann-Christine Syvänen et coll, décrivent, dans Nucleic Acids Research, vol. 16, 1988, p 11327-11338, une méthode pour mesurer la quantité d'ADN produit dans l'amplification dite par PCR. L'amplification est effectuée avec, en tant qu'amorces, des oligonucléotides modifiés en 5' par la biotine. On obtient ainsi des molécules ADN biotinylées en 5' qui sont détectées en solution par hybridation avec une sonde marquée, par exemple par le ³²P. Les hybrides formés sont capturés sur une matrice (particules de polystyrène) portant des groupes avidine, grâce à la réaction spécifique avidine-biotine.

Dans cette méthode, la fixation de l'avidine sur la matrice doit obligatoirement se faire de façon séparée, en faisant intervenir des réactions chimiques. De plus, la matrice ainsi modifiée ne peut être utilisée que pour capter des séquences biotinylées et ne permet donc l'analyse que d'une seule séquence nucléotidique dans chaque cas.

Yoshibo Nagata et coll. décrivent, dans FEBS Letters, vol. 183(2), 1985, p 379-382, la quantification de petites quantités d'ADN spécifique immobilisé dans des puits de plaques de microtitration en matière plastique. l'ADN dénaturé est immobilisé dans les puits par incubation suivie d'une irradiation. Il est ensuite hybridé avec une sonde ADN marquée à la biotine qui fixe ultérieurement, par réaction d'affinité spécifique, un complexe avidine-β-galactosidase. L'activité de l'enzyme ainsi retenue dans les puits est ensuite mesurée.

Ici, l'ADN peut être fixé directement sur un support parce qu'il présente une grande longueur (environ 50000 paires de bases). Au surplus, l'incubation est longue (une nuit) et l'immobilisation doit être assurée par une irradiation sous lampe à U.V.

Randall K, Saiki et coll, décrivent, dans Proc. Natl. Acad. Sci. USA, vol. 86, août 1989, p 6230-6234, une méthode pour cribler ou trier simultanément toutes les variantes alléliques connues d'un gène dans un échantillon.

Cette méthode utilise des filtres de nylon munis de sondes correspondant chacune à une variante allélique. Les sondes sont fixées sur les filtres par l'intermédiaire de "queues" homopolymères de désoxyribothymidine qui, sous irradiation UV, forment des liaisons covalentes avec le nylon. Le segment de gène concerné est amplifié par PCR au moyen d'amorces biotinylées puis est hybridé avec les sondes fixées sur le ou les filtre(s) de nylon. Les groupes biotine fixés sur le filtre par hybridation réagissent sélectivement avec la streptavidine d'un complexe streptavidine-peroxydase de raifort. La détection est effectuée colorimétriquement par réaction de la peroxydase sur un chromogène.

Ce procédé implique par conséquent, pour la fixation des sondes, l'utilisation de molécules longues et une irradiation. Il est donc relativement compliqué. De plus, on observe beaucoup de "bruit de fond", lors de la coloration par réaction enzymatique, comme cela est bien connu en ce qui concerne l'emploi de filtres en nylon auquel il est limité.

David J. Kemp et coll., décrivent, dans Proc. Natl. Acad. Sci., USA, vol. 86, avril 1989, p 2423-2427, un procédé de détection colorimétrique de segments d'ADN spécifiques, amplifiés par PCR qui utilise un support solide muni d'un complexe protéique contenant une protéine qui a une affinité spécifique pour une certaine séquence d'ADN double brin.

Dans ce procédé, l'ADN cible est d'abord amplifié par PCR en utilisant des amorces appropriées puis une seconde amplification est faite sur les molécules déjà amplifiées, en utilisant un autre couple d'amorces correspondant à des séquences situées à l'intérieur des molécules déjà amplifiées. Ces amorces portent chacune un ligand. Les deux ligands sont différents. L'un est, dans l' exemple donné, la biotine et l'autre une séquence d'ADN qui, lorsqu'elle est sous forme double brin, est reconnue par la protéine à affinité spécifique portée par le support.

L'ADN double brin résultant de ces amplifications et qui porte les deux ligands est fixé d'une part au support, par l'intermédiaire de son site spécifique de la protéine et d'autre part à un complexe avidine-enzyme par l'intermédiaire de la biotine. La détection colorimétrique se fait par réaction de l'enzyme, par exemple une peroxydase, sur un substrat chromogène.

Ce procédé qui implique deux séries d'amplifications par PCR est complexe et ne peut être utilisé que pour détecter des fragments double brins amplifiés.

R. Polsky - Cynkin et coll. décrivent, dans Clin. Chem. vol 31(9), 1985, p 1438-1443, l'utilisation d'ADN immobilisé sur des supports plastiques ou d'agarose pour détecter de l'ADN par hybridation de type sandwich. La fixation d'ADN sur le support est réalisée par revêtement de celui-ci au moyen d'un complexe protéine-ADN selon les "méthodes bien connues" des références 13 et 14 de cet article, à savoir l'article de G.H. Parsons Jr dans Methods Enzymol. vol 73, 1973, p 224-238 ("Antibody-coated plastic tubes in radioimmuoassay") et l'article de J. Welsh et coll. dans TIBS vol 9, 1984, p 505-508 ("Protein-DNA cross-linking"), respectivement. Aucune précision n'est donnée par R. Polsky-Cynkin et coll. quant au mode de réalisation exact de l'immobilisation de l'ADN mais la lecture des références citées permet de penser qu'un complexe réticulé protéine-ADN, préparé selon une des méthodes de la référence 14, est immobilisé sur un support, selon une des méthodes de la référence 13.

Cette immobilisation ne fait pas intervenir de réaction de type immunologique. Elle utilise seulement l'aptitude bien connue des supports en question à retenir les protéines par adsorption ou après traitement à l'aldéhyde glutarique.

Les molécules d'ADN utilisées par ces auteurs sont longues. Elles ont une longueur comprise entre 7600 et 15900 paires de bases.

Selon l'invention, on a maintenant eu l'idée d'utiliser la propriété de différents supports solides d'adsorber fortement les protéines, pour fixer des molécules d'acides ribo- ou désoxyribonucléiques d'une taille quelconque (en théorie, même un seul ribo- ou désoxyribonucléotide) sur un support, en associant un anticorps à l'acide nucléique, notamment un oligonucléotide, à fixer, par l'intermédiaire d'une réaction de type immunologique et d'utiliser le complexe immunologique formé comme moyen d'ancrage de l'acide nucléique sur le support choisi.

Il n'était pas évident au départ que le complexe anticorps-acide nucléique serait stable dans les conditions d'hybridation, ni que les oligonucléotides fixés par l'intermédiaire d'un anticorps pourraient conserver leur capacité de s'hybrider avec une séquence complémentaire.

Or, on a constaté que le système ou réactif ainsi obtenu non seulement était stable dans les conditions d'hybridation mais encore, comme il sera vu plus loin dans la partie expérimentale, ne modifiait pas la capacité de l'acide nucléique de s'hybrider avec une séquence spécifique complémentaire, permettant ainsi la détection, voire la détermination, de séquences nucléotidiques particulières dans un milieu d'essai complexe, et ceci même lorsque l'acide nucléique fixé sur le support ne comporte que quelques bases, c'est-à-dire est un oligonucléotide.

Au surplus, et cela est particulièrement surprenant, dans certains cas au moins, en particulier dans le cas de l'utilisation de supports solides de type filtre, une incubation préalable du mélange de l'anticorps et de l'acide nucléique muni d'une molécule pour laquelle cet anticorps a une affinité spécifique, n'est pas nécessaire.

Selon l'un de ses aspects, l'invention a pour objet un procédé de fixation d'une séquence nucléotidique sur un support solide par l'intermédiaire d'une protéine, caractérisé en ce que la protéine est un anticorps et en ce que la fixation fait intervenir l'adsorption de l'anticorps sur le support, d'une part et une réaction de type immunologique spécifique entre un substituant de la séquence nucléotidique, ou la séquence nucléotidique hybridée avec une molécule complémentaire, et ledit anticorps, d'autre part.

Le support solide en question est choisi parmi les supports capables d'adsorber fortement les protéines, en particulier les anticorps, sans modifier l'aptitude de ces derniers à donner des complexes immunologiques stables avec une molécule spécifique, notamment un haptène, d'une part et sans modifier de façon significative l'aptitude de la séquence nucléotidique fixée à s'apparier avec une séquence complémentaire se trouvant dans le milieu à étudier, d'autre part.

En tant que supports de ce type on peut citer, de façon non limitative, des filtres couramment utilisés pour les transferts par capillarité des protéines et des acides nucléiques, en particulier des filtres de nitrocellulose, de nylon ou de difluorure de polyvinylidène (PVDF) ou des matrices couramment utilisées dans les immunoessais, telles que des matières plastiques, par exemple le polypropylène ou le polystyrène, en particulier sous forme de tubes, ou des polyoses, notamment sous forme de "billes", tels que notamment celui commercialisé par la société Pharmacia Fine Chemicals sous la dénomination Sepharose®.

L'anticorps est en principe un anticorps spécifique d'un substituant porté par la séquence nucléotidique à fixer, avantageusement un anticorps monoclonal.

On peut toutefois imaginer, bien que cela ne présente a priori pas un grand intérêt, un système plus complexe faisant intervenir un anticorps "universel" qui fixerait lui-même l'anticorps spécifique, par exemple un anticorps anti-immunoglobulines ou une protéine ayant une affinité pour les anticorps.

La séquence nucléotidique fixée par ce procédé est une séquence d'ADN ou d'ARN, généralement couplée à un haptène tel que notamment la fluorescéine, le dinitrophényle (DNP), le N-acétoxy-N-2-acétylaminofluo-rène(AAF) ou son dérivé 7-iodo (AAIF), ou un groupe sulfone, et les anticorps spécifiques utilisés sont alors des anticorps anti-fluorescéine, anti-DNP, anti-guanosine AAF ou anti-cytosines sulfonées, respectivement.

Le couplage des ces haptènes avec la séquence nucléotidique, d'une part et la production des anticorps, notamment monoclonaux correspondants, d'autre part se font selon des méthodes bien connues de l'homme du métier.

De plus, l'haptène utilisé peut-être un groupement diméthoxytrityle (DMT) 5' terminal, introduit au cours de la synthèse de la séquence nucléotidique, en utilisant une séquence nucléotidique non déprotégée lors de la dernière étape de sa synthèse. L'anticorps utilisé est dans ce cas un anticorps anti-DMT.

Par ailleurs, dans le cas où la séquence à fixer est un hybride ARN/ADN (détection des virus à ARN ou des ARN messagers, ou détection d'ADNs avec des sondes ARN), les anticorps utilisés peuvent être des anticorps anti-hybrides ARN/ADN.

D'autres combinaisons "haptène"-anticorps spécifique peuvent facilement être imaginées par l'homme du métier sur la base de ses connaissances usuelles, sans sortir du cadre de l'invention.

La fixation de la séquence nucléotidique sur le support peut être réalisée en plusieurs étapes.

On peut ainsi adsorber d'abord l'anticorps sur le support poreux, dans les conditions bien connues décrites dans la littérature scientifique, puis incuber ensuite le système avec la séquence nucléotidique substituée par "l'haptène" correspondant, pour permettre la formation du complexe immunologique.

Il est toutefois préférable de mélanger d'abord l'anticorps et la séquence nucléotidique substituée ou hybridée pour former le complexe immunologique et d'adsorber ensuite l'ensemble sur un support solide. Il a en effet été constaté que dans ce cas, notamment lorsque l'on utilise des filtres, une incubation longue du mélange de la séquence nucléotidique substituée, notamment un oligonucléotide marqué par un haptène et de l'anticorps spécifique, n'était pas nécessaire et que ce mélange pouvait être filtré immédiatement, sans diminution du signal positif obtenu, comme cela est montré dans la partie expérimentale qui suit (détermination de la durée optimale d'incubation).

Cette constatation inattendue peut, semble-t-il, être attribuée au fait que la rencontre des anticorps et des haptènes fixés sur les séquences nucléotidiques est favorisée, et par conséquent leur réaction est accélérée, par le passage du mélange de ces molécules dans les pores du filtre. Cette propriété mérite d'être soulignée car elle est particulièrement intéressante pour les tests de diagnostic rapide.

Le procédé de fixation selon l'invention peut s'appliquer à la fixation de molécules d'acides ribo- ou désoxyribonucléiques de toute longueur, utilisés dans des réactions d'hybridation.

Comme indiqué plus haut, son utilisation est particulièrement intéressante dans le cas où la molécule à fixer est un oligonucléotide.

Il peut notamment être utilisé pour le typage d'agents pathogènes ou la caractérisation de mutations.

Dans ces utilisations, une sonde oligonucléotide ayant une affinité spécifique pour au moins une partie de la séquence à détecter est fixée sur un support solide selon le procédé de l'invention. La séquence à détecter, éventuellement amplifiée, est marquée par un moyen connu permettant, après lavage du support, sa mise en évidence directe, par exemple au moyen d'un marqueur radioactif tel que le ³²P, ou indirecte. Dans ce dernier cas, on peut utiliser des systèmes connus, plus ou moins complexes, d'identification dite " froide". A titre d'exemple, le marquage de la séquence à détecter peut être effectué par la biotine (comme décrit dans la partie expérimentale qui suit) et la mise en évidence de la séquence hybridée sur le support peut être réalisée au moyen d'un complexe avidine- ou streptavidine-enzyme, par action directe ou indirecte de l'enzyme sur un substrat tel qu'un chromogène ou un système de luminescence, selon des techniques connues de l'homme du métier en ce qui concerne les dosages immunoenzymatiques, notamment.

Selon une variante, la molécule à analyser, obtenue par exemple par PCR et portant un haptène peut être elle-même fixée sur le support par le procédé selon l'invention, en vue de sa détection ou de sa quantification par hybridation avec une sonde marquée, non liée au support.

Le procédé selon l'invention permet également de fixer sur un support solide, notamment poreux, des hybrides d'acides nucléiques, l'une au moins des molécules de l'hybride étant marquée en vue de sa détection. Les hybrides en question sont par exemple des hybrides ARN/ADN qui sont fixés par l'intermédiaire d'anticorps anti-hybrides ARN/ADN.

Le procédé selon l'invention peut en outre s'appliquer à la purification, par fixation sélective sur un support solide, notamment poreux, de molécules d'acides nucléiques synthétisées à partir d'une amorce marquée par un haptène, en utilisant pour la fixation un anticorps spécifique dudit haptène.

Selon un autre de ses aspects, l'invention vise un nécessaire ou kit pour la mise en oeuvre du procédé de fixation décrit ci-dessus. Ce nécessaire comprend au moins un support solide, notamment poreux et un anticorps, notamment monoclonal, spécifique du "haptène" servant à la fixation.

Dans le cas où la séquence nucléotidique à fixer est une sonde, le nécessaire comprend de plus, avantageusement, ladite sonde liée à l'haptène choisi.

Pour l'utilisation dans le cas où la séquence à analyser doit être amplifiée par PCR avant son hybridation sur la sonde fixée au support, le nécessaire comprend de plus, avantageusement, les amorces des extrémités de la séquence à amplifier, un moyen de marquage de cette séquence (par exemple du dUTP biotinylé), du dATP, du dTTP, du dGTP et du dCTP, ainsi que la polymérase utilisée pour la PCR.

Le nécessaire peut également comprendre les différents tampons et réactifs couramment utilisés dans la technique d'amplification par PCR, notamment tels que décrits dans la partie expérimentale qui suit.

Les conditions optimales de fixation dépendent essentiellement du support solide, de l'anticorps et de la séquence nucléotidique utilisés.

Elles peuvent être déterminées par des essais préalables, par exemple selon le protocole décrit dans la partie expérimentale qui suit.

A titre indicatif, pour la fixation d'un oligonucléotide combiné à la fluorescéine et comportant environ 20 bases, on peut déposer sur des filtres de nitrocellulose, de nylon ou de PVDF un mélange contenant de 0,5ng à 0,0625ng de l'oligonucléotide dans un volume final de 2 ml à 0,5 ml d'anticorps anti-fluorescéine dilués.

Par ailleurs, la quantité de cible fixée est avantageusement de l'ordre de 1ng.

La partie expérimentale qui suit est destinée à illustrer et mieux expliquer l'invention, sans toutefois limiter sa portée aux modes de réalisation plus particulièrement décrits.

### PARTIE EXPERIMENTALE

### I) Préparation des réactifs et essais préliminaires.

### A - Synthèse des oligonucléotides.

Des oligonucléotides comportant 15 à 25 bases et 3 groupements -NH ₂ 5' terminaux ont été synthétisés par des méthodes classiques en utilisant un synthétiseur Applied Biosystems. Pour chaque oligonucléotide, les groupements NH₂ 5' terminaux ont été incorporés au cours des 3 derniers cycles de la synthèse à l'aide de synthons "Aminomodifier II" commercialisés par Clontech, sans étapes de protection ("capping") entre ces trois cycles. Ainsi, chaque molécule synthétisée devait posséder entre 1 et 3 groupements NH₂ libres. Le rendement d'incorporation des synthons Aminomodifier II étant de 80% en moyenne, la proportion de molécules dépourvues de groupements NH₂ a été estimée à (0,2)³ = 0,008 soit 0,8%.

### B - Marquage des oligonucléotides :

### 1. Marquage en 5'.

Le ou les groupement(s) NH₂ 5' terminaux des oligonucléotides synthétiques ont été couplés à de la fluorescéine ou du dinitrophényle par réaction avec de l'isothiocyanate de fluorescéine (FITC) ou du 2,4 - dinitrofluorobenzène (réactif de Sanger), respectivement.

### a) - Couplage à la fluorescéine :

6,7 mg de FITC (Aldrich, référence F250-2) ont été dissous dans 0,34 ml de tampon bicarbonate de sodium 0,5M, (Na₂ CO₃ 0,5 M, 1 volume/ NaHCO₃ 0,5 M, 3 volumes). 17 µl de cette solution ont été mélangés à 20 µl d'une solution aqueuse de l'oligonucléotide à traiter à 5mg/ml. Après 12 heures d'incubation à 37°C, le mélange a été dialysé extensivement contre de l'eau distillée.

### b) - Couplage au dinitrophényle :

2 µl d'une solution aqueuse de l'oligonucléotide à traiter à 3 µg/µl ont été mélangés à 50µl de tampon de bicarbonate de sodium 0,2 M et 5 µl d'une solution de 2,4-dinitrofluorobenzène (Sigma, référence D6879) à 10% dans de l'éthanol. Après 12 heures d'incubations à 37°C, le mélange a été extrait 3 fois avec 300 µl d'éther froid puis dialysé extensivement contre de l'eau distillée.

### 2. Marquage en 3' par la biotine.

1µg d'oligonucléotide a été mis à réagir avec de la terminal désoxynucléotidyl transférase (Pharmacia, 10 unités), comme indiqué par Roychoudhury et Wu (Methods in Enzymology, vol.65, page 45, Academic Press, 1985), en présence de bio-11-dUTP (Gibco-BRL, 3 µl d'une solution 0,4 mM) dans un volume final de 20 µl. Le mélange a été incubé à 37° C pendant 16 heures, puis la réaction a été arrêtée par addition de 2 µl d'EDTA 50 mM, pH 7.

### C - Détermination des conditions optimales de fixation :

Un oligonucléotide de 20 bases marqué en 5' par la fluorescéine et en 3' par la biotine a été utilisé. Les mélanges d'oligonucléotides/anticorps anti-fluorescéine ont été filtrés à l'aide d'un appareil de filtration de type Hybridot ®(Gibco-BRL).

Dans une première expérience, l'anticorps a été dilué à 1/1000 dans du tampon citrate de sodium 0,015 M, pH 7/NaCl 0,15 M.

6 mélanges ont été faits :
1) - 0,5 ng d'oligonucléotide dans 2 ml d'anticorps
2) - 0,5 ng d'oligonucléotide dans 1 ml d'anticorps
3) - 0,25 ng d'oligonucléotide dans 1 ml d'anticorps
4) - 0,25 ng d'oligonucléotide dans 0,5 ml d'anticorps
5) - 0,125 ng d'oligonucléotide dans 0,5 ml d'anticorps
6) - 0,0625 ng d'oligonucléotide dans 0,5 ml d'anticorps
Les mélanges ont été incubés 1 heure à température ambiante puis filtrés. 3 types de filtres ont été essayés: nitrocellulose (BA83®, Schleicher et Schüll); nylon (POSIDYNE®, Pall); PVDF (IMMOBILON® P, Millipore).

Ce dernier filtre, hydrophobe a été mouillé avec du méthanol puis rincé à l'eau comme recommandé par son fabricant, avant filtration.

Après filtration, les filtres ont été séchés dans une étuve sèche à 80°C pendant 5 minutes puis saturés par incubation pendant 1 heure à température ambiante dans une solution de gélatine à 3% dans du citrate de sodium 0,015 M pH7/NaCl 0,15 M.

La présence de biotines a été détectée à l'aide d'un complexe phosphatase alcaline-streptavidine et d'un chromophore en utilisant un kit BluGene® (Gibco-BRL) et en suivant les indications du fabricant du kit.

Après une heure de révélation, toutes les taches étaient visibles. Les meilleurs résultats ont été obtenus avec les filtres de nitrocellulose qui ont été seuls utilisés pour les expériences suivantes.

### D - Détermination de la durée optimale d'incubation :

Des dépôts de 0,25 ng d'oligonucléotide dans 0,5 ml d'anticorps [mélange 4) ci-dessus] à une dilution de 1/1000 ont été faits après divers temps d'incubation à température ambiante (1 heure; 30 minutes; 10 minutes; sans incubation). Tous les dépôts ont donné des résultats identiques, et dans les expériences suivantes, les mélanges oligonucléotide/anticorps ont été filtrés sans incubation préalable.

### E - Vérification que la fixation dépend bien de l'anticorps spécifique de l'haptène couplé à l'oligonucléotide :

Deux échantillons du même oligonucléotide de 20 bases ont été marqués en 5' respectivement avec de la fluorescéine et du dinitrophényle, et en 3' avec de la biotine. Des aliquotes de 0,2 ng de ces oligonucléotides marqués ont été mélangées avec des anticorps anti-fluorescéine, anti-DNP ou du sérum de chèvre anti IgG de lapin, chaque antisérum étant à une dilution finale de 1/2000 dans du citrate de sodium 0,015M/NaCl 0,15M et le volume final du mélange étant de 0,5 ml.

De la même façon, des témoins du même oligonucléotide non marqué en 5' et biotinylé en 3' ont été mélangés aux trois types d'antisérums.

Après filtration et révélation des biotines, on a constaté que les oligonucléotides marqués à la fluorescéine sont retenus par les anticorps anti-fluorescéine et pas par les autres anticorps, les oligonucléotides marqués au DNP sont retenus par les anticorps anti-DNP uniquement et les oligonucléotides non marqués en 5'ne sont pas retenus.

De plus, les oligonucléotides marqués en 5' soit par la fluorescéine, soit par le DNP, filtrés en présence d'anticorps non spécifiques des marqueurs utilisés (sérum de chèvre anti-IgG de lapin) ne sont pas retenus.

### F - Détermination de la dilution optimale des anticorps anti-fluorescéine :

Une même quantité d'oligonucléotide marqué en 5' par la fluorescéine et en 3' par la biotine (0,2ng) a été filtrée avec 0,5 ml d'anticorps anti-fluorescéine à différentes dilutions (1/1000; 1/2000; 1/5000; 1/10000; 1/20000; 1/40000). Parallèlement, des volumes égaux (0,5 ml) d'anticorps à différentes dilutions (les mêmes que ci-dessus) ont été filtrés, puis les oligonucléotides (0,2 ng dans 0,5 ml du tampon citrate de sodium/NaCl décrit ci-dessus) ont été filtrés dans un deuxième temps, sans mélange préalable des anticorps avec les oligonucléotides.

Après révélation, on a constaté que les taches obtenues lorsque les oligonucléotides étaient mélangés aux anticorps avant filtration étaient plus intenses, et que l'intensité de ces taches diminuait à partir de la dilution de 1/10000. Pour les expériences suivantes, les anticorps anti-fluorescéine ont été utilisés à des dilutions variant entre 1/2000 et 1/5000.

### G - Détermination de la quantité optimale de cible et de la stabilité de la fixation :

Des quantités importantes (10ng; 5ng; 2ng; 1ng) d'oligonucléotides marqués en 5' par la fluorescéine et en 3' par la biotine ont été mélangées à une même quantité d'anticorps anti-fluorescéine (0,5 ml à une dilution 1/5000) puis filtrées.

Deux filtres ont été préparés dans les mêmes conditions avec les différentes quantités d'oligonucléotides indiqués ci-dessus. L'un a été incubé à 50° C pendant 16 heures dans une solution de préhybridation (voir plus loin) puis rincé 5 minutes dans du tampon citrate de sodium/NaCl à température ambiante avant coloration. La coloration s'est faite très rapidement dans les deux cas (moins de 5 minutes) et après 20 minutes il n'y avait pas de différence significative entre l'intensité des colorations.

Ainsi, il ne semble pas nécessaire de fixer des quantités d'oligonucléotide très supérieures à 1ng, et la fixation de l'oligonucléotide, obtenue par l'intermédiaire de l'anticorps, est suffisamment stable pour résister à des conditions normales d'hybridation.

### II) Applications :

L'utilisation de sondes oligonucléotidiques fixées par l'intermédiaire d'anticorps sur des filtres de nitrocellulose à été essayée dans un test de typage de papillomavirus humains (HPV).

Le principe du test est le suivant : des filtres portant des oligonucléotides dont les séquences sont spécifiques des types d'HPV à détecter sont mis en présence d'acides nucléiques marqués à la biotine de l'échantillon à analyser, dans des conditions permettant la formation de molécules hybrides. Après élimination des molécules biotinylées en excès, la présence des molécules biotinylées hybridées sur les oligonucléotides du filtre est détectée par une réaction enzymatique colorée.

Chaque sonde oligonucléotidique du filtre étant spécifique d'un type de virus, une coloration indiquant la présence d'un hybride stable sur cette sonde ne sera obtenue que si le type de virus correspondant est présent dans l'échantillon analysé.

Le marquage à la biotine des ADN de l'échantilllon à analyser est fait par incorporation de dUTP biotinylé lors d'une réaction d'amplification par PCR. Cette amplification par PCR utilise deux amorces spécifiques des HPV. L'une des amorces [amorce (+)] a une séquence commune à l'ensemble des types d'HPV étudiés, l'autre [amorce (-)] est spécifique de chaque type d'HPV.

Ainsi, le typage se fait avec un double contrôle : l'amplification, donc le marquage de la molécule qui s'hybridera sur la sonde, ne se fera que si le virus correspondant au type d'amorce utilisée est présent dans l'échantillon; l'apparition de taches colorées sur le filtre ne se fera que si des molécules amplifiées correspondant aux sondes oligonucléotidiques présentes sont obtenues avec les amorces utilisées pour la PCR.

### A - Préparation des filtres pour les hybridations.

Plusieurs filtres identiques de nitrocellulose (Schleicher et Schüll:BA 83) de 20 x 20mm ont été préparés avec quatre sondes oligonucléotidiques différentes. Les séquences des oligonucléotides utilisés, correspondant à des portions de génomes de papillomavirus humains (HPV) impliqués dans les cancers du col de l'utérus, sont les suivantes :
- séquence spécifique du virus de type 6 (HPV 6) :
   5' CAT CCG TAA CTA CAT CTT CC 3'
- séquence spécifique du virus de type 11 (HPV11) :
   5' CAT CTG TGT CTA AAT CTG CT 3'
- séquence spécifique du virus de type 16 (HPV 16) :
   5' GCC ATA TCT ACT TCA GAA AC 3'
- séquence spécifique du virus de type 18 (HPV18) :
   5' TCT ACA CAG TCT CCT GTA CC 3'
Chacun de ces oligonucléotides a été couplé en 5' à de la fluorescéine comme indiqué précédemment. Les dépôts sur les filtres ont été faits par filtration de 0,4 ml de citrate de sodium 0,015 M pH7/NaCl 0,015M contenant 4ng de l'oligonucléotide sonde choisi, couplé à la fluorescéine et des anticorps anti-fluorescéine à une dilution finale de 1/3500, comme indiqué précédemment. Après filtration des quatre types d'oligonucléotides, les filtres ont été séchés dans une étuve sèche à 80° C pendant 5 minutes puis incubés dans une solution de préhybridation (0,4% de Ficoll 400; 0,4% de polyvinylpyrrolidone 350; 0,4% de glycine; EDTA 40 mM; 0,8% de Tween®80; citrate de sodium 0,015M, pH7; NaCl 0,15M) pendant 7 heures.

Trois filtres identiques ont été ainsi préparés. Chacun de ces filtres a été hybridé avec une molécule biotinylée spécifique de l'une des sondes - respectivement HPV6, HPV16 et HPV18- comme indiqué ci-dessous.

### B - Préparation et hybridation de la molécule spécifique de HPV6 :

Un frottis cervical contenant un mélange de papillomavirus de type 6 (HPV6) et de type 16 (HPV16) a été utilisé comme source d'ADN viral. Les cellules du frottis ont été lysées dans un détergent (Triton^{®}X-100 à 1% dans de l'eau). Une portion de l'ADN viral comportant la séquence de la sonde a été amplifiée par la technique de "Polymerase Chain Reaction" (PCR).

Pour cela, une aliquote de 5 µl de cellules de frottis lysées a été utilisée pour un volume final de 50 µl de mélange réactionnel [KCl 50mM; Tris. HCl 10mM, pH 9; MgCl₂ 2,5mM; 0,01% de gélatine; 0,1% de Triton®X-100; dATP 0,2mM; dTTP 0,2mM; dGTP 0,2mM: dCTP 0,2mM; Bio11 dUTP 0,013mM; 75ng/50µl d'amorce (+); 75 ng/50µl d'amorce (-); 1 unité de Taq polymérase (Promega, EUA) pour 50µl].
Séquences des amorces utilisées :
amorce (+) :
5' TGT TTG TTA CTG TGG TAG ATA CCA CAC GCA GTA C 3'
amorce (-) :
5' CCC AAA AAC TAA GGT T 3'
(cette dernière amorce est spécifique de HPV6).

Les 50µl de mélange réactionnel ont été mis dans un tube Eppendorf de 1,5 ml et recouverts de deux gouttes d'huile de paraffine pour éviter l'évaporation.

Après une incubation de 2 minutes à 92°C, 40 cycles d'incubation de chacun une minute à 40°C, une minute à 72°C, une minute à 92°C ont été faits. Au dernier cycle, l'incubation à 92° C a été remplacée par une incubation de 10 minutes à 72°C. A la fin de cette amplification, 150 µl d'eau ont été ajoutés au tube et l'ensemble a été bouilli pendant 5 minutes puis refroidi rapidement dans de la glace.

Pour l'hybridation, un filtre préalablement soumis au traitement de préhybridation a été incubé pendant 16 heures à 46°C dans un mélange de 150µl de solution de préhybridation et 150µl de molécules biotinylées bouillies.

Après hybridation, le filtre a été rincé à température ambiante dans deux bains successifs (15 minutes dans du citrate de sodium 0,015M, pH7/NaCl 0,15M/0,2% de Tween®80 puis 5 minutes dans du citrate de sodium 0,003 M, pH7 NaCl 0,03M/0,2% de Tween® 80) et saturé pendant 1 heure dans une solution de gélatine à 3% dans du citrate de sodium 0,015M, pH7/NaCl 0,15M.

La présence de molécules biotinylées a été détectée à l'aide d'un kit BluGene (Gibco-BRL) en suivant les indications du fabricant du kit.

### C - Préparation et hybridation de la molécule spécifique de HPV 16 :

On a procédé comme pour la molécule spécifique de HPV6, si ce n'est que l'amorce (-) utilisée dans la PCR a été remplacée par l'amorce (-) spécifique de HPV16 qui a pour séquence
5' TCT TCT AGT GTG CCT CC 3'

### D - Préparation et hybridation de la molécule spécifique de HPV18 :

On a procédé comme ci-dessus, si ce n'est que des cellules de la lignée HeLa (Gey, G.O., et coll., Cancer Res., 12:264-265,1952), portant du virus HPV18 intégré, lysées dans du Triton®X-100 à 1% ont été utilisées à la place de cellules de frottis cervicaux. De plus, l'amorce (-) était l'amorce spécifique de HPV18 qui a pour séquence :
5' TC CTC TAA AAT ACT GCT 3'.

Après hybridation des trois filtres avec les molécules spécifiques de HPV6, HPV16 et HPV18, on a constaté que les taches colorées obtenues sur les filtres correspondaient bien aux types de virus utilisés.

## Revendications

1. Procédé de fixation d'une séquence nucléotidique sur un support poreux par l'intermédiaire d'un anticorps, faisant d'une part, intervenir l'adsorption de l'anticorps sur le support et d'autre part, une réaction de type immunologique spécifique entre un substituant de la séquence nucléotidique, ou la séquence nucléotidique hybridée avec une molécule complémentaire, et ledit anticorps, caractérisé en ce que l'on mélange d'abord une solution de l'anticorps et une solution de la séquence nucléotidique substituée ou hybridée et on fait passer ensuite le mélange, après incubation ou non, à travers le support poreux.

2. Procédé selon la revendication 1, caractérisé en ce que la séquence nucléotidique est un oligonucléotide.

3. Procédé selon la revendication 1 ou 2, caractérisé en ce que le support poreux est un filtre, de préférence un filtre de nitrocellulose, de nylon ou de difluorure de polyvinylidène.

4. Procédé selon l'une des revendications 1 à 3, caractérisé en ce que le substituant de la séquence nucléotidique est un haptène et en ce que l'anticorps est un anticorps spécifique de cet haptène, de préférence un anticorps monoclonal.

5. Procédé selon la revendication 4, caractérisé en ce que l'haptène est la fluorescéine, le DNP, l'AAF ou l'AAIF, un groupe sulfone ou un groupement diméthoxytrityle (DMT) et en ce que l'anticorps est un anticorps anti-fluorescéine, anti-DNP, anti-guanosine AAF, anti-cytosines sulfonées ou anti-DMT, respectivement.

6. Application du procédé selon l'une quelconque des revendications 1 à 5 au typage d'agents pathogènes ou à la caractérisation de mutations, de préférence après amplification enzymatique de type dit PCR de la ou les séquence(s) concernée(s).

7. Application du procédé selon l'une des revendications 1, 2 ou 3 à la fixation d'hybrides d'acides nucléiques dont l'une au moins des molécules est marquée en vue de sa détection, notamment des hybrides ARN/ADN, par l'intermédiaire d'anticorps anti-hybrides ARN/ADN.

8. Application du procédé selon l'une des revendications 1 à 5 à la purification, par fixation sélective sur un support poreux, de molécules d'acides nucléiques synthétisées à partir d'une amorce marquée par un haptène, en utilisant pour la fixation un anticorps spécifique dudit haptène.

9. Nécessaire ou kit pour la mise en oeuvre du procédé selon l'une des revendications 1 à 5, caractérisé en ce qu'il comprend au moins un support poreux et un anticorps, notamment monoclonal, spécifique du "haptène" servant à la fixation.

10. Nécessaire ou kit selon la revendication 9, caractérisé en ce qu'il comprend en outre une sonde nucléotidique liée à l'haptène choisi.

11. Nécessaire selon la revendication 9 ou 10 pour l'utilisation dans le cadre d'une analyse avec amplification de type dit PCR, caractérisé en ce qu'il comprend de plus les amorces des extrémités de la séquence à amplifier, un moyen de marquage de cette séquence, du dATP, du dTTP, du dGTP et du dCTP, ainsi que la polymérase utilisée pour la PCR.
